# EUROPEAN PATENT APPLICATION

(11) **EP 4 285 837 A1**
(43) Date of publication of application: **06.12.2023**
(21) Application number: 22305809.0
(22) Date of filing: 03.06.2022
(51) Int. Cl.: A61B 8/12, A61B 8/00

(54) **COMPACT ULTRASOUND TRANSDUCERS FOR INTRAORAL USES**

(71) Applicant: TROPHY SAS, 77435 Marne la Vallee Cedex 2 (FR); Carestream Dental LLC, Atlanta, GA 30339 (US); Université de Tours, 37020 Tours Cedex 1 (FR); Centre national de la recherche scientifique, 75016 Paris (FR); Institut National des Sciences Appliquées Centre Val de Loire, 18022 Bourges Cedex (FR); INSTITUT NATIONAL DE LA SANTE ET DE LA RECHERCHE MEDICALE - INSERM, 75013 Paris (FR)
(72) Inventor: CAPRI, Arnaud, 77435 MARNE LA VALLEE CEDEX 2 (FR); TOFFESSI SIEWE, Sean, 77435 MARNE LA VALLEE CEDEX 2 (FR); LEVASSORT, Franck, 37550 SAINT-AVERTIN (FR); CALLE, Samuel, 37270 LARÇAY (FR); GREGOIRE, Jean-Marc, 37390 METTRAY (FR)
(74) Representative: Santarelli

(57) **Abstract**

The present invention provides an ultrasound sensor for soft-tissue imaging comprising:
a piezoelectric element having a front side to be used for emitting ultrasound signals and for receiving echoed ultrasound signals to be measured and a back side; and
a backing element having a front side shaped to match the shape of the piezoelectric element back side, the backing element being made of a structured composite material comprising at least two base material, one of the two base material being a metal, the at least two base material having significant different acoustic impedance so that the backing element has an acoustic attenuation greater than 1.2 dB/mm/Mhz

## Description

### FIELD OF THE INVENTION

The present invention relates to the field of intraoral measurement devices for the health care industry. Particularly, but not exclusively, the invention relates to compact ultrasound transducers used in the dental industry for intraoral imaging applications.

### BACKGROUND OF THE INVENTION

Ultrasound imaging has been adapted for intraoral use in a number of implementations and has been found to have a particular utility for tasks such as measurement of periodontal pocket depth. Conditions such as gingivitis, for example, can be detected by sensing the acoustic response of tissues.

Because of the non-emission of ionizing radiation, ultrasound imaging is inherently safer than ionizing methods and also allows the repeatability of the examination if needed. Ultrasound imaging can be used as a substitute for, or a complement to, various types of radiography (cone beam computed tomography or CBCT, panoramic x-ray, or intraoral x-ray imaging), magnetic resonance imaging (MRI), or nuclear medicine.

Ultrasound imaging may use high-frequency sound waves, typically between 1 to 100 MHz. High-frequency waves being more attenuated than low-frequency waves for a given distance, high-frequency waves are suitable mainly for imaging superficial structures, e.g. for dermatology, or dental imaging. For example, high-frequency sound waves may preferably be between 20 to 50 MHz for periodontal pocket investigation. Conversely, low-frequency waves are suitable for imaging the deepest structures of the body. Moreover, image resolution is also improved when increasing frequency waves.

An ultrasound imaging apparatus generally comprises one or several transducers that acts as ultrasound beam emitters and/or ultrasound beam receivers to receive echoes from the emitted signals. In addition, the ultrasound imaging apparatus may comprise various processing and display components used for generating and presenting images from acquired signals. An ultrasound beam emitter generates an ultrasound signal from an electrical signal and conversely, an ultrasound receiver generates electrical pulses from a mechanical ultrasound signal.

Objects in the path of emitted ultrasound signals return a portion of the ultrasound energy back to the transducer which generates electrical signals indicative of the detected structures. Transducers can be of different types among single element transducers or multi-element transducers (annular array, linear array, 2D array, etc.). Furthermore, when a multi-element transducer is used, the emission of ultrasound signals can be delayed in order to enable adaptive focusing. The electronic adaptive focusing makes it possible to increase the resolution depending on the depth of the imaged organ. It also increases transducer sensitivity which improves contrast image.

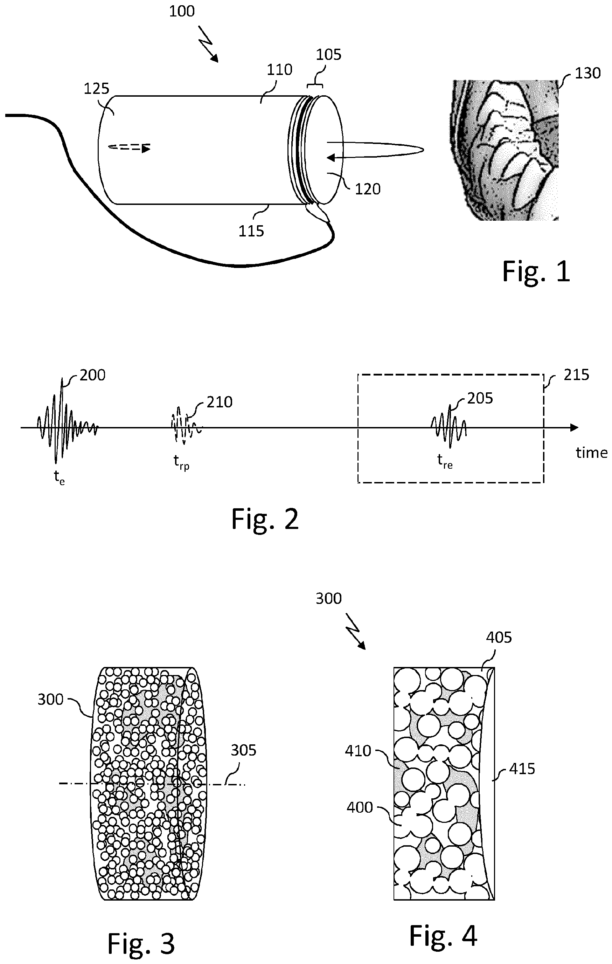

**Figure 1** is a schematic representation of a single element ultrasound transducer.

As illustrated, ultrasound transducer 100 comprises a piezoelectric element 105, acoustic material 110, also called a backing element, and a housing 115. It may also comprise an acoustic lens and matching layers (not represented) on the front face 120 of the piezoelectric element, to improve acoustic focus. The piezoelectric element typically comprises piezoelectric material arranged in between two electrodes that are connected to a pulse generator and receiver (not represented) through connection line 125. The piezoelectric material may convert electrical pulses into an ultrasound signal, when the electrodes are stimulated electrically, and may convert an ultrasound signal into an electrical signal. By processing, in a computing device (e.g. a personal computer) in signal communication with the ultrasound transducer, emitted ultrasound signals and measured echoed ultrasound signals (as illustrated by the solid arrow), it is possible to generate images characterizing the object reflecting the emitted ultrasound signal, for example gums and other intraoral soft tissues (and possibly tooth surfaces), generically referenced 130. The piezoelectric element may be, for example, a poly(vinylidene fluoride - poly(vinylidene fluoride-trifluoroethylene (P(VDF-TrFE)) piezoelectric film.

It is noted that acoustic material or backing element 110 aims at damping piezoelectric element 105 (from a mechanic point of view) and at attenuating ultrasound signals generated on the back face of piezoelectric element 105 (as illustrated by the dotted arrow), in order to reduce parasitic echoes that may perturb the measures performed on the front face of the transducer. To avoid any perturbation of the measures performed on the front face of the transducer, the back side echoed signals should be received by the piezoelectric element after the front side echoed signals. In addition, the back side echoed signals should be fully absorbed or their amplitude should be reduced as much as possible. In view of the material generally used for manufacturing the backing elements, this leads to backing elements of significant sizes.

**Figure 2** illustrates an example of ultrasound signals emitted and received by an ultrasound transducer over time, with a low attenuation backing. For the sake of illustration and in a simplified way, it is assumed that a short acoustic signal is emitted at time tₑ in one direction, for example by exciting briefly, with an electrical pulse, the piezoelectric element between the two electrodes of ultrasound transducer 100 in Figure 1. This emitted signal, referenced 200, is reflected by the object to be imaged, for example gums and other intraoral soft tissues (and possibly tooth surfaces) 130 in Figure 1. The echoed acoustic signal, referenced 205, is received at time tᵣₑ by the transducer. Depending on the objects to be imaged, multiple echoes may be produced at different times, constituting a Radio Frequency (RF) line or echo line in one direction. As illustrated, an additional parasitic signal (reference 210), resulting from an ultrasound signal emitted on the back side of the piezoelectric element, is reflected within the backing element, for example within backing element 110 in Figure 1. It contributes to the RF line and is received by the transducer at time tᵣₚ. As illustrated, the characteristics of the backing element are such that the signal echoed in the backing element reaches the piezoelectric element significantly sooner than the signal to be measured (i.e. the signal echoed by the object to be imaged) so as to avoid any perturbation of the measures.

To improve measures, the acquisition of echoed signals is generally done during acquisition time windows, referenced 215, that are defined as a function of the characteristics of the transducer, of the object to be imaged, and of the estimated distance between the transducer and the object to be managed. By setting appropriately the acquisition windows and using a transducer having an efficient backing element, effects of parasitic echoes are minimized. Nevertheless, it is to be noted that multiple parasitic echoes often superimpose on the echo line of interest within acquisition time windows 215.

While such ultrasound transducers are generally efficient, particular challenges with intraoral ultrasound imaging relate to the design of a probe that can be used for imaging a full set of intraoral structures, i.e. for positioning an ultrasound transducer along the vertical axis of each tooth of a mouth on both buccal and lingual faces, in view of the size of the transducers. Indeed, to be efficient, the ultrasound transducer must be facing the regions to be imaged.

Therefore, there is a need for improving ultrasound transducers to improve apparatus for ultrasound imaging of gums and other intraoral soft tissues (and possibly of tooth surfaces), to make them more compact and cheaper.

### SUMMARY OF THE INVENTION

The present invention has been devised to address one or more of the foregoing concerns.

In this context, there is provided a compact ultrasonic transducer adapted for ultrasound imaging of gums and other intraoral soft tissues (and possibly of tooth surfaces).

According to an aspect of the invention, there is provided an ultrasound transducer for soft-tissue imaging comprising:
a piezoelectric element having a front side to be used for emitting ultrasound signals and for receiving echoed ultrasound signals to be measured and a back side; and
a backing element having a front side shaped to match the shape of the piezoelectric element back side, the backing element being made of a structured composite material comprising at least two base material, one of the two base material being a metal, the at least two base material having significant different acoustic impedance so that the backing element has an acoustic attenuation greater than 1.2 dB/mm/Mhz.

The compact ultrasound transducer according to the invention makes it possible to integrate easily the transducer within an intraoral probe that can be used for imaging a full set of intraoral structures, i.e. for positioning an ultrasound transducer along the vertical axis of each tooth of a mouth on both buccal and lingual faces. In particular, the compact ultrasound transducer of the invention makes it possible to measure the depth of periodontal pockets from ultrasound images.

In an embodiment, the backing element has an acoustic impedance greater than 25 MPa.s/m.

In an embodiment, the acoustic impedance of the other of the at least two base materials is equal to or less than half the acoustic impedance of the one base material.

In an embodiment, the one base material comprises sintered metal balls, for example sintered bronze.

In an embodiment, the diameter of the metal balls is comprised between half the wavelength value of the emitted ultrasound signal to twice the wavelength value of the emitted ultrasound signal.

In an embodiment, the other base material is tin.

In an embodiment, the piezoelectric element is of the P(VDF-TrFE) type.

In an embodiment, the thickness of the backing element is less than 3 mm, preferably less than 2 mm, along the longitudinal axis of the transducer.

In an embodiment, the shape of the backing element back side, opposite to the back side of the piezoelectric element, is concave, convex, or conic.

According to another aspect of the invention, there is provided a method for manufacturing the ultrasound transducer described above, the method comprising,
- obtaining a porous piece made of the one base material, and
- filing pores of the porous piece with the other base material.

In an embodiment, the method further comprises machining the filed porous material to shape the backing element, coating the backing element front side with nickel, and/or selecting the one base material, the one base material being selected as a function of a backing element target thickness, an acoustic impedance, and/or an acoustic attenuation.

According to still another aspect of the invention, there is provided an intraoral dental probe comprising the ultrasound transducer described above.

### BRIEF DESCRIPTION OF THE DRAWINGS

Embodiments of the invention will now be described, by way of example only, and with reference to the following drawings in which:
**Figure 1** is a schematic representation of a single element ultrasound transducer;
**Figure 2** illustrates an example of ultrasound signals emitted and received by an ultrasound transducer over time;
**Figure 3** illustrates an example of a backing element of an ultrasound transducer, according to some embodiments of the invention;
**Figure 4** illustrates a sectional view of the backing element illustrated in Figure 3, along a longitudinal plane;
**Figure 5** illustrates examples of the shape of the backing element back side, opposite the backing element side receiving a piezoelectric element; and
**Figure 6** illustrates an example of steps for manufacturing a backing element according to some embodiments of the invention.

### DETAILED DESCRIPTION OF THE INVENTION

The following is a detailed description of particular embodiments of the invention, reference being made to the drawings in which the same reference numerals identify the same elements of structure in each of the Figures.

In the drawings and text that follow, like components are designated with like reference numerals, and similar descriptions concerning components and an arrangement or interaction of components already described are omitted. Where they are used, the terms "first", "second", and so on, do not necessarily denote any ordinal or priority relation, but may simply be used to more clearly distinguish one element from another, unless specified otherwise.

In the context of the present disclosure, the terms "viewer", "operator", and "user" are considered to be equivalent and refer to the viewing practitioner, technician, or other person who acquires, views, and manipulates an ultrasound image, such as a intraoral image, on a display monitor. An "operator instruction," "user instruction," or "viewer instruction" is obtained from explicit commands entered by the viewer, such as by clicking a button on the ultrasound probe or system hardware or by using a computer mouse or by using a touch screen or a keyboard entry.

In the context of the present disclosure, the phrase "in signal communication" indicates that two or more devices and/or components are capable of communicating with each other via signals that travel over some type of signal path. Signal communication may be wired or wireless. The signals may be communication, power, data, or energy signals. The signal paths may include physical, electrical, magnetic, electromagnetic, optical, wired, and/or wireless connections between the first device and/or component and second device and/or component. The signal paths may also include additional devices and/or components between the first device and/or component and second device and/or component.

The term "subject" refers to the gums and other intraoral soft tissues (and possibly to tooth surfaces) of a patient that is being imaged and, in optical terms, can be considered equivalent to the "object" of the corresponding imaging system.

According to some embodiments of the inventions, a backing element having a high acoustic impedance and made of a structured composite material is used to cause a scattering effect on the ultrasound signals emitted on the back side of a piezoelectric element while improving the sensibility of the ultrasound transducer. Such a scattering effect mainly consists in dividing an ultrasound signal into a plurality of ultrasound sub-signals that are in turn divided into pluralities of ultrasound sub-signals, and so on. This scattering effect allows reducing both the amplitude and the coherence of the ultrasound sub-signals reflected back to the piezoelectric element. The backing element is suitable for intraoral ultrasonic investigations with a high acoustic impedance compared with the piezoelectric element acoustical impedance (e.g. suitable backing acoustic impedance should be greater than 25 MPa.s/m or MRayl, more preferably greater than 30 MPa.s/m for a piezoelectric element acoustic impedance of the order of 4.5 MPa.s/m) in order to improve the sensitivity of transducers. The acoustic impedance of the backing element is preferably at least 5 times higher than the acoustic impedance of the piezoelectric element. The frequency behavior of the backing element on the transducer element is such that the transducer central frequency is preferably between 20 to 50 MHz. In addition, the backing element has a high attenuation (e.g. greater than 1.2 dB/mm/MHz) in order to avoid or reduce backside echoes due to the mechanical wave generation.

In other words, the backing element is heavy enough (i.e. it has a high acoustic impedance with regard to the piezoelectric element acoustic impedance) to allow strong energy transmission from the piezoelectric element toward tissues to be imaged, comprises many internal diffraction surfaces to attenuate the backside effect of the mechanical wave generation, and has a size that is compatible with intraoral ultrasonic investigations. For the sake of illustration, the backing element may have a thickness less than 3 mm, preferably 2 mm, along a longitudinal axis of the piezoelectric element. Still for the sake of illustration, it may have a diameter less than 1 cm, for example a diameter of 6 mm.

It is noted here that ultrasound transducers based on piezoelectric copolymer P(VDF-TrFE) are known for their relatively low sensitivity due to their low effective thickness coupling factor (denoted kₜ) compared to other common piezoelectric materials such as PZT. The acoustic impedance of the P(VDF-TrFE) is typically of the order 4.5 MPa.s/m (MRayl). Piezoelectric copolymer P(VDF-TrFE) material is suitable for high frequency ultrasound applications and is easily conformable (due to its flexibility) compared to ferroelectric ceramics of PZT type.

Using a backing element having an acoustic impedance lower than or close to 4.5 MPa.s/m (light backing), in conjunction with a piezoelectric element of the P(VDF-TrFE) type, leads to a preference for bandwidth (120%) of the transducer at the expense of sensitivity. This sensitivity can be significantly improved using a backing with a high acoustical impedance (heavy backing). For an acoustic impedance of 80 MPa.s/m, sensitivity can increase of 7 dB compared to previous configuration comprising an air backing, with a relative bandwidth of 60%. The inventors have determined that using a backing element with a high acoustic impedance is suitable. For the sake of illustration, the inventors have determined that a backing element having an acoustic impedance of 32 MPa.s/m allows to improve the sensitivity of 3dB with a relative bandwidth of 72% compared to the reference configuration comprising an air backing. This result is an excellent trade-off between sensitivity and bandwidth in imaging applications.

It is also to be noted that while the material of the backing element should have a high acoustic impedance value, it should be easily conformable, for example to form a front face cup for the geometric focusing of the transducer. The inventors have observed that some metal substrates, for example sintered bronze (i.e. an alloy of copper and tin), could be used as backing materials for piezoelectric polymers due to the conformable characteristic and high acoustic impedance.

In addition, the material of the backing element should have a high acoustic attenuation in order to avoid backside echoes, with a thickness compatible with probe specifications (e.g. a thickness of the backing element that is less than 3 mm, preferably less than 2 mm). According to some embodiments of the invention, such a high acoustic attenuation value is reached by using the scattering effect of a multiphasic medium, such as a multiphasic medium based on bronze, tin, and air, forming a controlled micro-porous material. An efficient scattering effect may be obtained when using a metal ball shaped base material, the ball (or grain) size being of the same order as the wavelength value of the excitation signal (e.g. it is comprised in the range from half the wavelength value of the excitation signal to twice the wavelength value of the excitation signal). For example, bronze grains having an average size of 100 µm may be used to attenuate an ultrasound signal having a wavelength within the range [80 µm; 220 µm] corresponding to frequency range [20MHz; 50MHz]. Presence of tin and/or air in a bronze ball structure reduces interferences due to the large acoustical impedance mismatch.

Some measurements have shown a high acoustic attenuation of a backing element having a thickness of 1.7 mm, made from sintered bronze grains having an average size of 100 µm, wherein space between the sintered grains have been partially filed by tin (i.e. there remains space between the sintered grains that is filed by air). Measurements carried out on different samples have led to an acoustic attenuation average value of 34.7 dB/mm for an ultrasound signal having a frequency of 25 MHz.

Figure 3 illustrates an example of a backing element of an ultrasound transducer, according to some embodiments of the invention. As illustrated, backing element 300 has a cylindrical shape with one concave face to match the shape of a piezoelectric element (not represented). It is made of sintered bronze, the gap between the bronze grains being filed with tin and air. Such a structure provides scattering effects onto ultrasound signals, that result from the structure itself and from the ratio of the acoustic impedance between bronze on the one hand, and tin and air on the other hand. As mentioned above, backing element 300 may have a thickness less than 3 mm, preferably 2 mm, along a longitudinal axis of the piezoelectric element (reference 305). It may also have a diameter less than 1 cm, for example a diameter of 6 mm.

**Figure 4** illustrates a sectional view of the backing element illustrated in Figure 3, along a longitudinal plane, showing its structure comprising sintered bronze grains 400, air 405, and tin 410. It also shows concave shape portion 415 that is suitable for receiving a piezoelectric element. Of course, other structures and materials may be used.

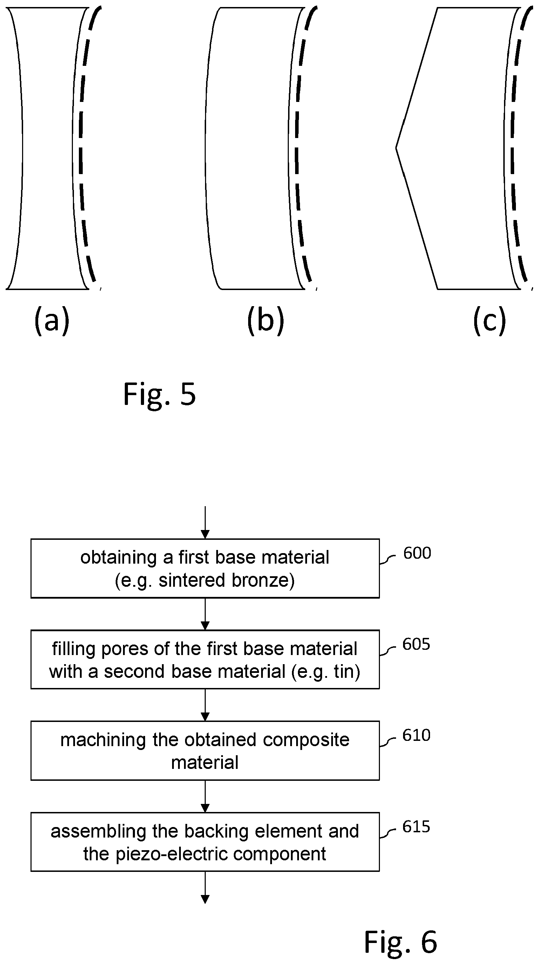

It is observed that the backing element side opposite the side receiving the piezoelectric element may be of different shapes than the one illustrated, to reflect received ultrasound signals in directions other than the longitudinal direction of the backing element, which result is increasing the scattering effects of the backing element, as illustrated in Figure 5.

**Figure 5** illustrates examples of the shape of the backing element back side, opposite the backing element side receiving a piezoelectric element (represented with dotted lines).

As illustrated, such a shape may be concave (a), convex (b), or conical (c). It may also be a combination of different shapes (not represented).

**Figure 6** illustrates an example of steps for manufacturing a backing element according to some embodiments of the invention.

As illustrated, a first step (step 600) is directed to obtaining a first base material having a first acoustic impedance, such as sintered bronze (dense bronze having an acoustic impedance of about 40 to 45 MPa.s/m). For the sake of illustration, such sintered bronze material may be obtained from the Ames company (e.g. AmesPores^{®}). According to some embodiments, the first base material consists of bronze spherical particles that are compacted in a mold and then sintered. The mold may be arranged to take into account the shape of the piezoelectric element that will be mounted on the backing element. Alternatively, the obtained material is machined later on to adjust the front face to the shape of the piezoelectric element.

Sintering may consist in heating the compacted bronze spherical particles, without melting the particles (for bronze, the melting temperature varies from 700° C to 1 300° C, depending on the alloy composition). Under the effect of heat, the grains weld together and lead to the cohesion of the particles. As a result, a metal part with a controlled level of micro-porosity is obtained. According to some embodiments, sintered bronze slices having an average grain size of 100 µm, an outside diameter of 8 mm, and a thickness of 10 mm are obtained. They may be obtained directly from sintered bronze cylinders that are cut (at this stage or later on) to obtain slices. Still according to some embodiments, the porosity rate of the obtained slices is 35% with an average pore size of 53 µm (and with a maximum size of 139 µm), the grain and pore size of these slices being therefore consistent with the wavelength values of the ultrasound signals to attenuate (e.g. around 80 µm to 220 µm).

Such first base material is preferably a structured material, that is to say that it arranged according to a given geometric structure making it porous. In addition, the first material is preferably homogenous, meaning that the density of the first base material is approximately constant within the different portions of the slices. Such structured and homogenous material may be referred to as a structured material skeleton.

These slices may be used directly as backing elements (possibly after machining to adjust the size, polishing, etc.). However, it has been observed that their porosities make it difficult to bound piezoelectric elements, in particular P(VDF-TrFE) piezoelectric elements, directly on such slices. Accordingly, impregnation of the slices, or of one or more portions of the slices with a second base material is preferably carried out (step 605).

The second base material should be chosen as a function of its own characteristics but also of the characteristics of the first base material. In particular, the second base material should be chosen as a function of its acoustic impedance and as a function of the acoustic impedance of the first base material. According to some embodiments, the acoustic impedance difference between the second base material and the first base material is suitable to induce diffraction effect while the acoustic impedance of the backing element remains high compared to the piezoelectric element impedance. For the sake of illustration, the acoustic impedance of the first base material may be about twice the acoustic impedance of the second base material. Still for the sake of example, tin may be chosen as the second base material since it is a ductile metal with the low melting temperature (232° C), making possible the impregnation with materials having high melting temperatures like bronze (890° C). The penetration of tin occurs at temperatures around 400° C, which may be done by using a soldering iron. In addition, tin have an acoustic impedance of about 20 MPa.s/m that is very different from the one of the bronze, about half of the bronze impedance value (40 MPa.s/m), leading to a composition having a significant scattering effect.

According to particular embodiments, impregnating the first base material with the second base material comprises introducing a sintered bronze slice or cylinder into a tinning flux (e.g. the LOCTITE HYDX-20 Liquid Flux) and degassing the assembly for a given time, for example for 5 min. The flux cleans the material to improve the adhesion of the tin. Then, tin may be introduced within the pores of the bronze slice or cylinder, for example by using a soldering iron positioned on the surface of the bronze slice or cylinder, enabling tin penetrating inside the bronze slice or cylinder by a capillarity effect. The tin used may be a lead-free alloy (e.g. Sn 96% Ag 4% SAC305]) having a melting temperature of 232° C.

According to another embodiment, epoxy resin is used to fill partially or totally the pores of the bronze slice or cylinder. To that end, a bronze slice or cylinder (cut later to obtain slices) may be surrounded by an adhesive comprising openings and bathed in an epoxy resin under vacuum conditions. During vacuum degassing of the slice or cylinder, the air contained within the slice or cylinder is replaced by the epoxy resin. Then, the assembly is heated, for example at 65° C for one hour, for polymerizing the epoxy resin.

Next, the slices are machined (possibly after being obtained from cylinders). For the sake of illustration, machining may comprise adjusting the size of the slices to the size of the backing elements (e.g. reducing the thickness and/or the diameter of the slices), conforming the front face of the slices to the shape of the piezoelectric elements with which the backing element should be mounted, cleaning, for example cleaning with an abrasive product, and polishing the backing elements. Machining may also comprise coating the backing element, for example coating the front side of the backing element (i.e. the side against which the piezoelectric element is to be mounted) with nickel to avoid formation of whiskers over time, that may create parasitic signals.

Optionally, the obtained material (e.g. bronze slice or cylinder) can be further pressed with an element having a convex portion (e.g. a ball) to create a concave shape portion on the face to be assembled onto the piezoelectric element, the concave shape being adapted to focus the ultrasonic beam (it being noted that a P(VDF-TrFE) piezoelectric element is flexible).

In addition, it has been observed that filing the gap between the bronze grains with tin or another material that comes solid after being added, at least where the piezoelectric element is to be assembled, improves the quality of the surface where the piezoelectric element is to be assembled and thus, improves bonding of the piezoelectric element on the backing element, without affecting the electroacoustic properties of the transducer.

Next, a backing element and a piezoelectric element are assembled (step 615), possibly with an acoustic lens, and mounted within a housing. For the sake of illustration, the piezoelectric element may be glued onto the backing element using an epoxy resin. it is noted that the adhesive layer must be thin enough not to interfere with the expected operation of the transducer.

Although the present disclosure has been described herein above with reference to some specific embodiments (e.g. single element transducer), the present invention is not limited to these specific embodiments, and modifications will be apparent to a person skilled in the art which lie within the scope of the present invention. In particular, the present invention can be used for multi-element transducers such as linear transducers, annular arrays of transducers, or 2D arrays of transducers. The shape of the backing element is preferably adapted accordingly (e.g., it may take a parallelepipedic shape). In addition, the shape of the backing element surface to which the piezoelectric element is glued may be different from the piezoelectric element surface glued to the backing element (e.g, one surface may have a square shape while the other may have a circle shape). Likewise, the shape of the sintered bronze material from which is obtained the backing element may be non-cylindrical.

Many further modifications and variations will suggest themselves to those versed in the art upon making reference to the foregoing illustrative embodiments, which are given by way of example only and which are not intended to limit the scope of the invention, that being determined solely by the appended claims. In particular, the different features from different embodiments may be interchanged, where appropriate.

In the claims, the word "comprising" does not exclude other elements or steps, and the indefinite article "a" or "an" does not exclude a plurality. The mere fact that different features are recited in mutually different dependent claims does not indicate that a combination of these features cannot be advantageously used.

## Claims

1. An ultrasound transducer for soft-tissue imaging comprising:
a piezoelectric element having a front side to be used for emitting ultrasound signals and for receiving echoed ultrasound signals to be measured and a back side; and
a backing element having a front side shaped to match the shape of the piezoelectric element back side, the backing element being made of a structured composite material comprising at least two base material, one of the two base material being a metal, the at least two base material having significant different acoustic impedance so that the backing element has an acoustic attenuation greater than 1.2 dB/mm/Mhz.

2. The ultrasound transducer according to claim 1, wherein the backing element has an acoustic impedance greater than 25 MPa.s/m.

3. The ultrasound transducer according to claim 1 or claim 2, wherein the acoustic impedance of the other of the at least two base materials is equal to or less than half the acoustic impedance of the one base material.

4. The ultrasound transducer according to any one of claims 1 to 3, wherein the one base material comprises sintered metal balls.

5. The ultrasound transducer according to claim 4, wherein the diameter of the metal balls is comprised between half the wavelength value of the emitted ultrasound signal to twice the wavelength value of the emitted ultrasound signal.

6. The ultrasound transducer according to any one of claims 1 to 5, wherein the one base material is sintered bronze.

7. The ultrasound transducer according to any one of claims 1 to 6, wherein the other base material is tin.

8. The ultrasound transducer according to any one of claims 1 to 7, wherein the piezoelectric element is of the P(VDF-TrFE) type.

9. The ultrasound transducer according to any one of claims 1 to 8, wherein the thickness of the backing element is less than 3 mm, preferably less than 2 mm, along the longitudinal axis of the transducer.

10. The ultrasound transducer according to any one of claims 1 to 9, wherein the shape of the backing element back side, opposite to the back side of the piezoelectric element, is concave, convex, or conic.

11. A method for manufacturing the ultrasound transducer according to any one of claims 1 to 10, the method comprising,
- obtaining a porous piece made of the one base material, and
- filing pores of the porous piece with the other base material.

12. The method of claim 11, further comprising machining the filed porous material to shape the backing element.

13. The method of claim 11 or claim 12, further comprising coating the backing element front side with nickel.

14. The method of any one of claims 11 to 13, further comprising selecting the one base material, the one base material being selected as a function of a backing element target thickness, an acoustic impedance, and/or an acoustic attenuation.

15. An intraoral dental probe comprising an ultrasound transducer according to any one of claims 1 to 10.
